(19) Europäisches Patentamt
European Patent Office
Office européen des brevets

(11) Numéro de publication: **0 100 935 B1**

# (12) FASCICULE DE BREVET EUROPEEN

(45) Date de publication du fascicule du brevet: **26.02.86**

(51) Int. Cl.⁴: **C 07 D 307/94**, C 07 D 311/96, C 11 B 9/00 // C07C35/18

(21) Numéro de dépôt: **83107101.4**

(22) Date de dépôt: **20.07.83**

(54) **Composés spiro-lactoniques nouveaux, leur utilisation en tant qu'agents parfumants, composition parfumante les contenant et procédé pour leur préparation.**

(30) Priorité: **12.08.82 CH 4835/82**

(43) Date de publication de la demande: **22.02.84 Bulletin 84/8**

(45) Mention de la délivrance du brevet: **26.02.86 Bulletin 86/9**

(84) Etats contractants désignés: **CH DE FR GB LI NL**

(56) Documents cités:
**FR - A - 2 405 251**

**BULLETIN DE LA SOCIETE CHIMIQUE DE FRANCE, 1961, pages 1509-1512, Paris, FR., M. MOUSSERON-CANET et al.: "Isomérisation photochimique dans la série de l'alpha-ionone"**
**CHEMICAL ABSTRACTS, vol. 96, no. 25, 21 juin 1982, page 767, no. 218067f, Columbus, Ohio, USA**

(73) Titulaire: **FIRMENICH SA, 1, route des Jeunes, CH-1211 Genève 8 (CH)**

(72) Inventeur: **Giersch, Wolfgang Klaus, 323, rue de Bernex, CH-1233 Bernex (CH)**
Inventeur: **Ohloff, Günther, Dr., 13, Chemin de la Chapelle, CH-1233 Bernex (CH)**

(74) Mandataire: **Salvadori, Giuseppe, Dr., c/o Firmenich S.A. Case Postale 239, CH-1211 Genève 8 (CH)**



Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1986

## Description

La présente invention a trait au domaine de la parfumerie; elle concerne plus particulièrement l'utilisation en tant qu'agent parfumant d'un composé spiro-lactonique de formule I

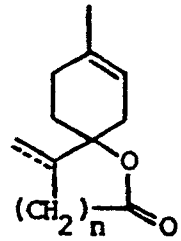

(I)

pouvant posséder une liaison supplémentaire à la position indiquée par les pointillés et dans laquelle l'indice n' vaut 1 ou. 2. Il s'agit plus particulièrement de la 5,9-diméthyl-l-oxaspiro[5.5]undéc-8-ène-2-one, de la 9-méthyl-5-méthylène-loxaspiro[5.5] undéc-8-ène-2-one et de la 4,8-diméthyl-l-oxaspiro[4.5] déc-7-ène-2-one, composés de structure nouvelle.

Nous avons découvert que lesdits composés possédaient des propriétés odorantes fort intéressantes et, de ce fait, pouvaient être employés avantageusement pour le parfumage d'articles divers, tels les savons, les produits cosmétiques, les shampoings, les poudres à lessive ou les produits d'entretien par exemple, ou pour la manufacture de parfums, bases parfumantes ou concentrés, ainsi que pour la reconstitution d'huiles essentielles. Les composés de formule I conviennent également au parfumage d'adoucisseurs ou de revitalisants textiles.

La 9-méthyl-5-méthylène-loxaspiro[5.5]undéc-8-ène-2-one, ainsi que la 4,8-diméthyl-l-oxaspiro[4.5]déc-7-ène-2-one, possèdent une note odorante épicée particulièrement riche qui s'apparente à celle développée par la coumarine. Ce caractère est tout à fait inédit en parfumerie et la valeur des composés de formule I est d'autant plus grande que l'on cherche à remplacer la coumarine dans certaines de ses applications.

La 5,9-diméthyl-l-oxaspiro[5.5]undéc-8-ène-2-one possède par contre un caractère olfactif typiquement boisé qui rapelle celui de l'essence de cèdre.

Les proportions dans lesquelles les composés de l'invention peuvent être utilisés pour produire les effets désirés varient dans une gamme de valeurs assez étendue. Lors de la manufacture de bases ou concentrés, des concentrations préférentielles sont comprises entre environ 2-3% et 20%, parties en poids, tandis que, bien entendu, lors du parfumage d'articles tels les savons, les produits cosmétiques ou capillaires, les produits d'entretien ou les détergents, des concentrations préférentielles sont de l'ordre de 0,2% à 1% en poids du produit terminé. L'utilisateur peut par ailleurs varier à son gré la valeur des proportions en deça ou au-delà des valeurs indiquées, en fonction des effets spécifiques qu'il désire obtenir ou en fonction de la nature des produits qu'il désire parfumer.

Les composés de l'invention peuvent être utilisés à l'état isolé par adjonction directe aux articles à parfumer ou, de préférence, sous forme d'une solution et en mélange avec d'autres ingrédients parfumants dans une composition donnée. La variété des dissolvants et des adjuvants est telle que l'homme de l'art pourra opérer son choix en se référant sans autre aux ouvrages classiques de la parfumerie et aux exemples reportés par l'art antérieur.

Comme il a été indiqué plus haut, les composés de l'invention sont des entités chimiques nouvelles. Ils sont obtenus conformément à la présente invention par un procédé qui consiste en

a. l'addition d'oxyde d'éthylène sur du p-mentha-1,8-diène-4-ol en présence d'une base forte, et

b. l'oxydation au moyen de bichromate de pyridinium du diol ainsi obtenu de formule II

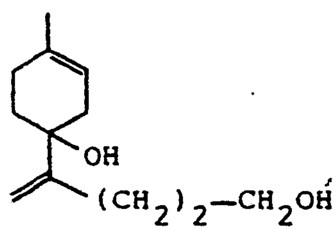

(II)

pour fournir la 9-méthyl-5-méthylène-loxaspiro[5.5]undéc-8-ène-2-one; ou

b'. la réduction du diol II pour fournir le diol de formule III

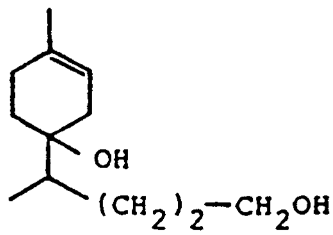

(III)

et

c". l'oxydation de ce dernier au moyen de bichromate de pyridinium pour donner la 5,9-diméthyl-1-oxaspiro[5.5]undec-8-ène-2-one, ou

b". l'hydroformylation du p-mentha-1,8-diène-4-ol pour donner le composé de formule

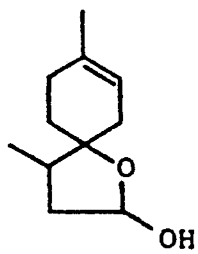

et

c". le traitement de ce dernier composé à l'aide de bichromate de pyridinium pour fournir la 4,8-diméthyl-1-oxaspiro[4.5]déc-7-ène-2-one.

Le procédé indiqué ci-dessus est illustré à l'aide du schéma réactionnel suivant:

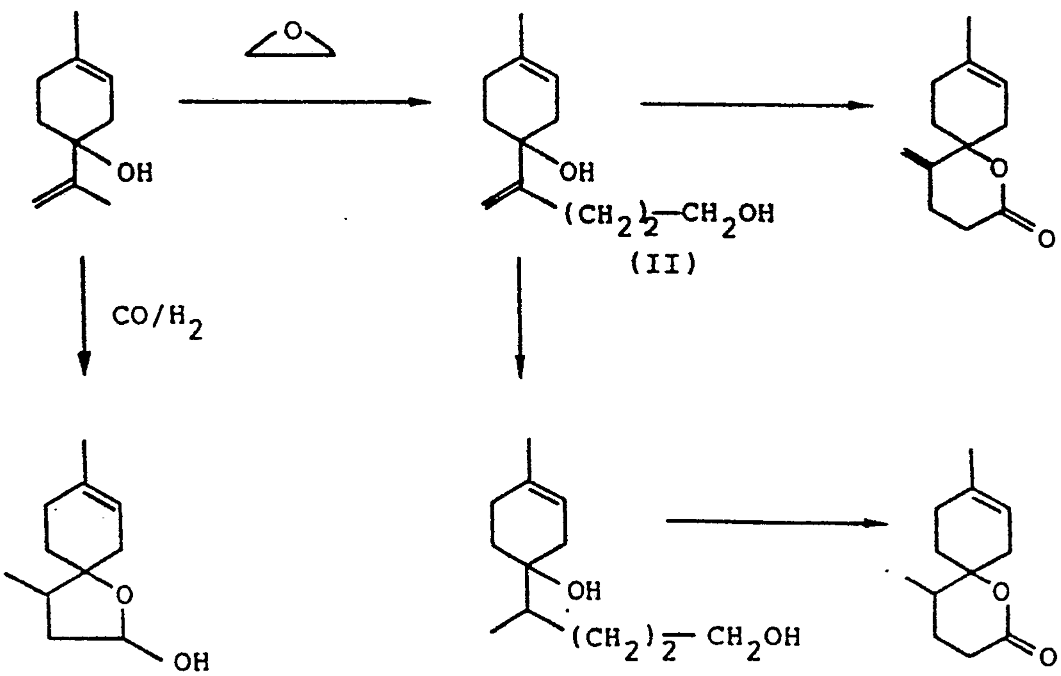


(II)

(III)

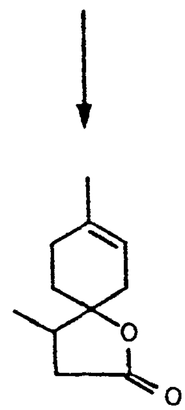

L'invention est définie comme indiqué aux revendications et est illustrée de manière plus détaillée dans les exemples qui suivent. Dans lesdits exemples, les températures sont indiquées en degrés centigrades et les abréviations ont le sens usuel dans l'art.

**Exemple 1**

9-Methyl-5-methylene-1-oxaspiro[5,5]undéc-8-ène-2-one

a. 4-[1-hydroxy-4-méthyl-cyclohex-3-ène-1-yl]pent-4-ène-1-ol

551 g (4,75 M) de N,N,N',N'-tétraméthyl-éthylène-diamine (TMEDA) ont été ajoutés goutte à goutte à température ambiante à 2130 g (4,75 M) d'une solution à 15% dans l'hexane de butyl-lithium. L'addition s'accompagne d'un léger réchauffement du mélange réactionnel (45°). Après 30 minutes à cette température, on a refroidi à 0° et l'on y a ajouté ensuite 345 g de p-mentha-1,8-diène-4-ol dans 500 ml d'éther anhydre et le tout a été maintenu à 0° pendant une nuit. La masse brunâtre ainsi formée a été dissoute dans 500 ml de THF anhydre puis la solution résultante a été refroidie à -50°. A cette température on a ajouté 100 g (2,27 M) d'oxyde d'éthylène durant 5 h, puis, une fois l'addition terminée, la température du mélange a été portée à température ambiante et l'agitation a été maiantenue pendant une nuit.

L'hydrolyse du mélange a été effectuée à l'aide d'une solution concentrée de $NH_4Cl$. La phase organique a été lavée avec une solution concentrée de NaCl jusqu'à neutralité et, après évaporation, le résidu a été distille sur une colonne de type Vigreux de 15 cm de longueur. On a ainsi obtenu 177 g du diol désiré ayant une pureté d'environ 90% (rendement 63% basé sur le p-mentha-1.8-diène-4-ol de départ transformé). Eb. 130-145° /1,33 Pa. IR: 3350, 3033, 1645, 910 $cm^{-1}$

SM: M+ = 196(2); m/z: 178(8), 163(3), 111(67), 95(67), 84(49), 69(100), 55(73), 41(87).

b. 9-méthyl-5-méthylène-1-oxaspiro[5.5]undec-8-ène-2-one

Un mélange constitué par 140 g (0,38 M) de bichromate de pyridinium, 16,5 g (0,084 M) du diol obtenu comme indiqué au paragraphe précédent, et 600 ml de $CH_2Cl_2$ a été maintenu sous agitation à température ambiante pendant 18h. Un mélange de 300 ml d'éther diéthylique et 300 ml d'éther de pétrole (30/50) a ensuite été ajouté et le mélange résultant a été filtré. Le filtrat clair a été évaporé sous vide et le résidu distillé à l'aide d'un appareil à boules (température du bain: 150°). On a ainsi obtenu 10,3 g (rendement 42%) d'un produit ayant une pureté de 65% environ.

IR ($CDCl_3$): 1730 $cm^{-1}$;

SM: M+ = 192 (35); m/z: 177(3), 164(8), 132(18), 124(35), 96(100), 81(19), 68(40), 55(32), 41(40);

RMN (60 MHz): 1,71 (3H, s); 2,00-2,06 (4H, 2s); 2,44 (2H,t); 2,63 (4H,s); 5,03 et 5,10 (2H,d); 5,33 (1H) σ ppm.

Le p-mentha-1,8-diène-4-ol, utilisé comme produit de départ dans le procédé décrit ci-dessus, peut être obtenu suivant le procédé décrit par E. Girandi et al., Recherches, 19, 205 (1974).

**Exemple 2**

5,9-Diméthyl-1-oxaspiro[5.5]undec-8-ène-2-one

a. 6,4 g au diol obtenu suivant la méthode décrite au paragraphe a. de l'exemple 1 ci-dessus, dans 70 ml

d'acétate d'éthyle ont été hydrogénés en présence de palladium sur charbon. Après adsorption de 860 ml d'hydrogène, le mélange a été filtré et le filtrat clair a été évaporé pour fournir 6,2 g d'une huile noirâtre.

b. Un mélange constitué par 6,0 g (0,03 M) du diol obtenu comme indiqué sous lettre a. ci-dessus, 40 g de bichromate de pyridinium et 250 ml de diméthylformamide a été maintenu sous agitation et atmosphère d'azote pendant 16 h, puis il a été dilué avec de l'eau et extrait avec plusieurs portions de $CH_2Cl_2$. Les extraits réunis ont été lavés avec du NaCl saturé, séchés et évaporés.

Par distillation à l'aide d'un four à boules on a obtenu 3,15 g d'une huile orange (température du bain: 150° environ; 1,33 Pa) dont les caractères analytiques d'un échantillon purifié étaient les suivants:

IR: 1735 $cm^{-1}$;

RMN (360 MHz): 1,025 et 1,04 (3H, 2d); 1,68 (3H, s); 5,26 (1H, s) σ ppm;

SM: M+ = 194(60); m/z: 143(63), 121(34), 98(100), 93(90), 68(55), 55(60), 41(47).

**Exemple 3**

4,8-Diméthyl-l-oxaspiro[4.5]déc-7-ène-2-one

a. 4,8-Diméthyl-1-oxaspiro[4.5]déc-7-ène-2-ol

60 G de p-mentha-1,8-diène-4-ol, 0,09 g de $HRhCO(PPh_3)_3$ et 300 ml de cyclohexane ont été laissés réagir pendant 4 h à 90° dans une atmosphère de CO et $H_2$ à environ 200 bar dans un autoclave. Après évaporation des parties volatiles, sous pression réduite, on a distillé le résidu pour fournir une fraction à Eb. 90-112°/4 Pa; 63,5 g. Ce produit a été soumis à une distillation fractionnée pour donner un produit pur.

IR: 3420 $cm^1$.

5 G (27,5 mM) du produit obtenu sous lettre a. ci-dessus ont été traités avec 11,3 g (30 mM) de bichromate de pyridinium dans 100 ml de chlorure de méthylène. Le tout a été maintenu 48 h sous agitation à température

b. 4,8-Diméthyl-l-oxaspiro[4.5]déc-7-ène-2-one

5 G (27,5 mM) du proauit obtenu sous lettre a. ci-dessus ont été traités avec 11,3 g (30 mM) de bichromate de pyridinium dans 100 ml de chlorure de méthylène. Le tout a été maintenu 48 h sous agitation à température ambiante, puis le mélange a été filtré et le filtrat clair a été évaporé et distillé dans un appareil à boules.

Eb. 120° (bain)/1,33 Pa; 5g.

IR: 1770$cm^{-1}$;

RMN: 1,05 (3H, d, J=6); 1,68 (3H, large s); 5,3 (1H, large s) σ ppm;

SM: M+ = 180; m/z: 162 (2), 120 (15), 112 (33), 93 (32),68 (100), 42(63).

**Exemple 4**

On a procédé au parfumage d'un shampoing à base protéinique à l'aide de 0,3 et 0,5% en poids de 9-méthyl-5-méthylène-1-oxaspiro[5.5]undéc-8-ène-2-one. Le produit résultant possédait un caractère odorant plaisant, la note désagréable de la base était ainsi masquée.

**Exemple 5**

On a préparé une composition parfumante de type lavande en mélangeant les ingrédients suivants (parties en poids):

Essence de cannelle de Ceylan à 10%* 200

| | |
|---|---|
| Essence de lavande | 180 |
| Essence de géranium Bourbon | 100 |
| Acétate de vétyvéryle | 60 |
| Essence de girofle | 60 |
| Cédrène | 80 |
| Acétate de linalyle | 40 |
| Benzoate de benzyle | 60 |
| Galbanum gomme 25%* | 20 |
| Essence de bergamote synth. | 20 |
| Musc DTI[1)4)] | 20 |
| Exaltex [1)3)] | 20 |
| Tricyclone[1)2)] | 20 |
| Isoeugenol | 10 |
| Essence de patchouli | 10 |
| | 900 |

* dans le phtalate diéthylique
1) origine Firmenich SA, Genève
2) voir brevet CH 626'532; 10,10-diméthyltricyclo[7.1.1.$0^{2,7}$]undéc-2-ène-4-one
3) pentadécanolide
4) 1,1-dimethyl-4-acétyl-6-tert-butylindane

En ajoutant à la composition de base ainsi obtenue de la 9-méthyl-5-méthylène-loxaspiro[5.5]undéc-8-ène-2-one à raison de 10%, on obtient une nouvelle composition dont le caractère olfactif était plus riche, plus élégant et harmonieux. La note épicée était en outre plus complexe, sans posséder pour autant l'aggressivité montrée par l'addition, à la même composition de base, d'une proportion identique de coumarine.

Lorsqu'on ajoute à raison de 10% à la composition de base ci-dessus indiquée un mélange constitué par des quantités équivalentes de coumarine et de 9-méthyl-5-méthyléne-l-oxaspiro[5.5]undéc-8-ène-2-one , l'on obtient une nouvelle composition parfumante dont le caractère olfactif est préféré à celui contenant l'un ou l'autre des deux ingrédients pris isolément. Le composé spiro-lactonique de l'invention se marie donc fort bien avec la coumarine et sert à rehausser le caractère épicé de cette dernière.

**Revendications**

1. Composé spiro-lactonique de formule I

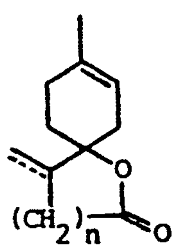

(I)

pouvant posséder une liaison supplémentaire à la position indiquée par les pointillés et dans laquelle l'indice n vaut 1 ou 2.

2. 5,9-Dimethyl-l-oxaspiro[5.5]undéc-8-ène-2-one.

3. 9-Méthyl-5-méthylène-l-oxaspiro[5.5]undéc-8-ène-2-one.

4. 4,8-Dimethyl-l-oxaspiro[4.5]déc-7-ène-2-one.

5. Utilisation d'un composé spiro-lactonique de formule I suivant la revendication 1 en tant qu'agent parfumant.

6. Composition parfumante résultant de l'utilisation suivant la revendication 5.

7. Procédé pour la préparation d'un composé spiro-lactonique suivant la revendication 1, caractérisé en ce qu'on

a. additionne l'oxyde d'éthylène sur du p-mentha-1,8-diène-4-ol en présence d'une base forte, et

b. oxyde au moyen de bichromate de pyridinium le diol ainsi obtenu de formule II

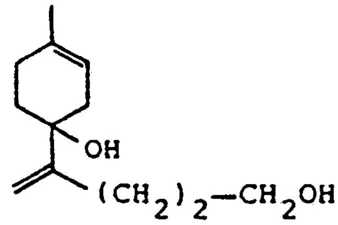

(II)

pour fournir la 9-méthyl-5-méthylène-loxaspiro[5.5]undéc-8-ène-2-one; ou

b'. réduit le diol II pour fournir le diol de formule III

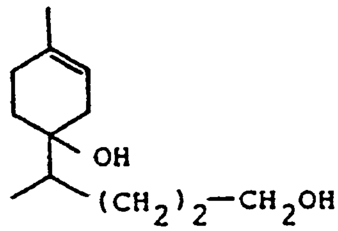

(III)

et

c'. oxyde ce dernier au moyen de bichromate de pyridinium pour donner la 5,9-diméthyl-1-oxaspiro[5.5]undéc-8-ène-2-one, ou

b". hydroformyle le p-mentha-1,8-diène-4-ol pour donner le composé de formule

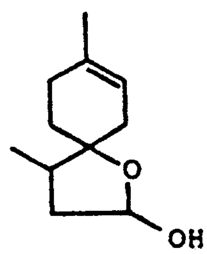

et

c". traite ce dernier composé à l'aide de bichromate de pyridinium pour fournir la 4,8-diméthyl-l-oxaspiro[4.5]déc-7-ène-2-one.

**Patentansprüche**

1. Spirolacton der Formel I

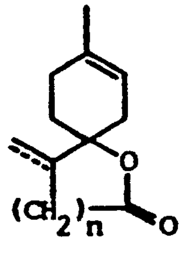

(I)

das in der durch die gestrichelte Linie bezeichneten Stellung eine zusätzliche Bindung enthält und in welcher der Index n 1 oder 2 darstellt.

2. 5,9-Dimethyl-l-oxaspiro[5.5]undec-8-en-2-on.

3. 9-Methyl-5-methylen-l-oxaspiro[5.5]undec-8-en-2-on.

4. 4,8-Dimethyl-1-oxaspiro[4.5]dec-7-en-2-on.

5. Verwendung eines Spirolactons der Formel I gemäss Anspruch 1, als Riechstoff.

6. Riechstoffkomposition, die durch die Verwendung gemäss Anspruch 5 hergestellt wird.

7. Verfahren zur Herstellung eines Spirolactons gemäss Anspruch 1, dadurch gekennzeichnet, dass man
a. Ethylenoxyd an p-Mentha-1,8-dien-4-ol in Anwesenheit einer starken Base addiert, und
b. das so erhaltene Diol der Formel II

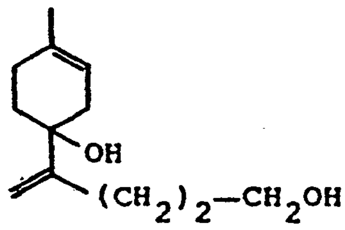

(II)

mittels Pyridinium Bichromat oxydiert, um 9-Methyl-5-methylen-l-oxaspiro[5.5]undec-8-en-2-on zu erhalten; oder
b'. das Diol II reduziert um Diol III

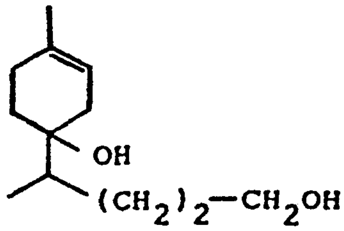

(III)

zu erhalten, und
c'. das letztere mittels Pyridinium Bichromate oxydiert, um 5,9-Dimethyl-l-oxaspiro[5.5]undec-8-en-2-on zu erhalten, oder
b". p-Mentha-1,8-dien-4-ol hydroformyliert, um die Verbindung der Formel

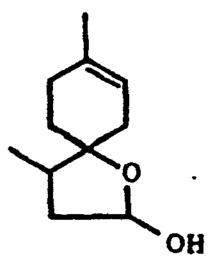

zu erhalten, und
c". das letztere mittels Pyridinium Bichromate behandelt, um 4,8-Dimethyl-l-oxaspiro[4.5]dec-7-en-2-on zu erhalten.

**Claims**

1. Spirolactone of formula I

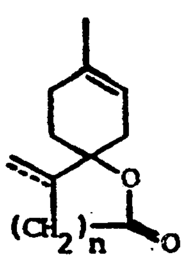

(I) .

which can possess a supplementary bond at the position indicated by the dotted line and wherein index n stands for 1 or 2.

2. 5,9-Dimethyl-l-oxaspiro[5,5]undec-8-en-2-one.
3. 9-Methyl-5-methylene-l-oxaspiro[5.5]undec-8-en-2-one.
4. 4,8-Dimethyl-l-oxaspiro[4.5]dec-7-en-2-one.
5. Utilization of a spirolactone of formula I according to claim 1 as perfuming agent.
6. Perfume composition resulting from the utilization according to claim 5.
7. Process for the preparation of a spirolactone according to claim 1, characterized in that

a. ethylene oxide is added to p-mentha-1,8-dien-4-ol in the presence of a strong base, and
b. the resulting diol of formula II is oxidized by means of pyridinium bichromate

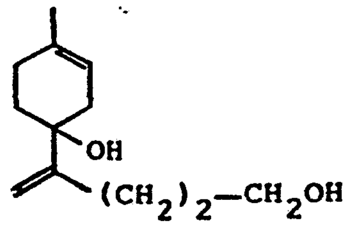

(II)

to give 9-methyl-5-methylene-l-oxaspiro[5.5]undec-8-en-2-one; or
b'. diol II is reduced to give a diol of formula III

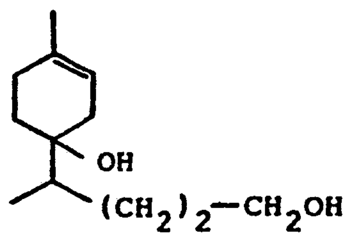

(III)

and
c'. this latter compound is oxidized by means of pyridinium bichromate to give 5,9-dimethyl-l-oxaspiro[5.5]undec-8-en-2-one, or
b". p-mentha-1,8-dien-4-ol is hydroformylated to give a compound of formula

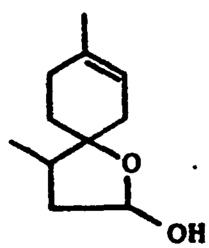

and
c". this latter compound is treated with pyridinium bichromate to give 4,8-dimethyl-1-oxaspiro[4.5]dec-7-en-2-one.